# EUROPEAN PATENT APPLICATION

(11) **EP 3 572 379 A1**
(43) Date of publication of application: **27.11.2019**
(21) Application number: 18209938.2
(22) Date of filing: 03.12.2018
(51) Int. Cl.: C02F 1/68, A61Q 19/00, A61Q 19/10, B01D 24/00, C02F 1/28, C02F 1/42, C02F 103/02

(54) **BEAUTY WATER GENERATOR WITH HIGH PERFORMANCE FILTERING FUNCTION**

(30) Priority: 23.05.2018 KR 20180058588
(71) Applicant: Korea New Tech Co., Ltd., Daejeon 34036 (KR)
(72) Inventor: Jung, Ji Haeng, 34036 Daejeon, (KR)
(74) Representative: Gulde & Partner

(57) **Abstract**

The present invention relates to a beauty water generator (100) with a high performance filtering function, the beauty water generator (100) including: a housing portion (110) used as a body of the beauty water generator (100); a mounting portion (200) provided inside the housing portion (110); and a filter unit (300) having a first filter part (300a) that purifies introduced raw water through bottom-up movement and discharges purified water and a second filter part (300b) that, when the purified water is introduced therein, imparts functionality to the purified water through bottom-up movement and discharges beauty water, and provided inside the housing portion (110) such that the first filter part (300a) and the second filter part (300b) are mounted and removed by rotating through the mounting portion (200).

## Description

### CROSS REFERENCE TO RELATED APPLICATION

The present application claims priority to Korean Patent Application No. 10-2018-0058588, filed May 23, 2018, the entire contents of which is incorporated herein for all purposes by this reference.

### BACKGROUND OF THE INVENTION

### Field of the Invention

The present invention relates to a beauty water generator with a high performance filtering function. More particularly, the present invention relates to a beauty water generator with a high performance filtering function, the beauty water generator not only providing beauty water with various functions such as a filtering function using multiple filters and moisturizing function but also maintaining the performance.

### Description of the Related Art

As it is well known, due to the increasing interest in the beauty of the skin, various skin beautifying products have been released. Accordingly, beauty water generators have been developed, which generate beauty water and provide beauty water for washing face.

A beauty water generator performs filtering or softening of household tap water (that is, raw water) such that water is suitable for use on sensitive skin, adds cosmetic materials that help the skin in treated water, and provides treated water to users.

For example, there has been proposed a beauty water generator in which a cosmetic material is filled in a cosmetic filter, and water is passed through the cosmetic filter such that beauty water is generated. However, the beauty water generator has a problem in that a fragrance or cosmetic effect is continuously reduced as a certain time elapses after the first use, and thus the effects cannot be provided any longer.

Accordingly, as mentioned above, beauty water generators have been researched and developed, which provide beauty water continuously with various and desired beauty functions.

### Documents of Related Art

(Patent Document 1) Korean Utility Model No. 20-0438980, issued on March 7, 2008; and
(Patent Document 2) Korean Patent No. 10-0484922, issued on April 13, 2005

### SUMMARY OF THE INVENTION

Accordingly, the present invention has been made keeping in mind the above problems occurring in the related art, and an objective of the present invention is intended to provide a beauty water generator with a high performance filtering function, the beauty water generator not only providing beauty water with various functions such as a filtering function using multiple filters and a moisturizing function but also maintaining the performance thereof.

In addition, another objective of the present invention is to provide a beauty water generator with a high performance filtering function, the beauty water generator purifying water introduced into a filter container by using multiple filters while guiding the water from the bottom to the top, and imparting various functionalities (for example, skin health, moisturizing, natural preservative, antibacterial, etc.) to water, and discharging the water to the top such that it is possible to perform filtering by using the multiple filters and effectively impart a moisturizing function and additional health functionalities to water and supply the water.

In addition, another objective of the present invention is to provide a beauty water generator with a high performance filtering function, the beauty water generator having at least one filter container having multiple filters inside a housing and easily mounting and removing the filter container, such that it is possible to replace an existing filter container with a filter container having multiple filters having various functions and supply beauty water having various functionalities.

Furthermore, another objective of the present invention is to provide a beauty water generator with a high performance filtering function, the beauty water generator having a relief valve provided at a lower portion of a filter container to regulate an inner pressure and having filters provided inside the filter container and spaced apart a predetermined distance from each other, such that it is possible to maintain the filtering function and the moisturizing function and supply beauty water.

The objectives of the present invention will not be limited only to the objectives described above. Accordingly, additional objectives of the present invention will be set forth in part in the description which follows and in part will become apparent to those having ordinary skill in the art upon examination of the following or may be learned from practice of the present invention.

In order to achieve the above object, according to one aspect of the present invention, there is provided a beauty water generator with a high performance filtering function, the beauty water generator including: a housing portion used as a body of the beauty water generator; a mounting portion provided inside the housing portion; and a filter unit having a first filter part that purifies introduced raw water through bottom-up movement and discharges purified water and a second filter part that, when the purified water is introduced therein, imparts functionality to the purified water through bottom-up movement and discharges beauty water, the filter unit being provided inside the housing portion such that the first filter part and the second filter part are mounted and removed by rotating through the mounting portion.

According to the embodiment of the present invention, the housing portion may include: a housing body having an accommodating portion therein to accommodate the mounting portion and the filter unit; a first inflow passage through which the raw water is introduced into the first filter part; a first outflow passage through which the purified water is discharged from the first filter part; a second inflow passage through which the purified water is introduced into the second filter part; and a second outflow passage through which the beauty water is discharged from the second filter part.

According to the embodiment of the present invention, the mounting portion may be provided in a manner that corresponds to the first filter section and the second filter section, and the mounting portion may include: a first support member fixed to an upper portion of the accommodating portion; a second support member engaged with the first support member to have a space for axial rotation; a rotary shaft cylinder provided in the space for the rotational axis and having an outflow passage at an outlet end, which corresponds to an inflow passage formed inside an inlet end; and a mounting cap which is rotatably engaged with the rotary shaft cylinder and on which the filter unit is mounted in a rotationally engaged manner.

According to the embodiment of the present invention, the first filter part may include: a cylindrical first filter housing; a first inner inflow passage transferring the raw water to a lower portion along an inner side surface of the first filter housing; a first filter container provided inside the first filter housing to accommodate the first filter; a first-first filter provided inside the first filter container and having multiple sediment filters stacked therein; a first-second filter provided on the first-first filter and made of an adsorbent material; a first-third filter provided on the first-second filter and having multiple stacked ion exchange filters (IE filters); a first inner outflow passage discharging the purified water purified through the first-first filter, the first-second filter, and the first-third filter to the outside of the first filter part; and multiple first spacers spacing the first-first filter, the first-second filter, and the first-third filter apart from each other by a predetermined distance.

According to the embodiment of the present invention, the first filter part may further include: a first relief valve at a lower end of the first filter housing, which discharges an inner fluid to maintain a predetermined pressure of the first filter part.

According to the embodiment of the present invention, the multiple sediment filters may have different pore sizes.

According to the embodiment of the present invention, the first-first filter may be disposed such that the multiple sediment filters provided inside a first-first filter housing are spaced a predetermined distance apart from each other.

According to the embodiment of the present invention, the adsorbent material may include at least one of activated carbon, zeolite, loess, goethite, and illite.

According to the embodiment of the present invention, the first-second filter may be provided inside a first-second filter housing as a disc-shaped filter made of the adsorbent material and spaced a predetermined distance apart from an inner surface of the first-second filter housing.

According to the embodiment of the present invention, each of the multiple IE filters may have a functional module, and the functional module is formed in a disc shape, has an ion exchange function (IE), and has functional fibers and filtration fibers being alternately arranged.

According to the embodiment of the present invention, the multiple IE filters of the first-third filter may be stacked inside a first-third filter housing and arranged to be spaced a predetermined distance apart from each other, and a residual spacer may be provided in each portion, which is adjacent to the multiple IE filters.

According to the embodiment of the present invention, the second filter part may include: a cylindrical second filter housing; a second inner inflow passage transferring the purified water to a lower portion along an inner side surface of the second filter housing; a second filter container provided inside the second filter housing to accommodate the second filter; multiple second filters provided inside the second filter container and imparting at least one function of moisturizing, aroma, vitamin, and antiseptic and antibacterial to the purified water; and a second inner outflow passage discharging the beauty water treated through the multiple second filters to the outside of the second filter part.

According to the embodiment of the present invention, the second filter part may further include: a second relief valve at a lower end of the second filter housing, which discharges an inner fluid to maintain a predetermined pressure of the second filter part.

According to the embodiment of the present invention, the multiple second filters may include at least one functional filter selected from the group consisting of a vitamin infusing filter, a moisturizing filter, and an antiseptic and antibacterial filter. The vitamin infusing filter may include vitamin selected from the group consisting of vitamin A, vitamin B, vitamin C, and vitamin E, the moisturizing filter may include a material selected from the group consisting of red ginseng, hempseed oil, honey extract, lotus oil, and camellia oil, and the antiseptic and antibacterial filter may include a material selected from the group consisting of peppermint, lemongrass, chitin, and chamomile.

The present invention provides a beauty water generator with a high performance filtering function, the beauty water generator not only providing beauty water with various functions such as a filtering function using multiple filters and a moisturizing function but also maintaining the performance thereof.

In addition, the present invention provides a beauty water generator purifying water introduced into a filter container by using multiple filters while guiding the water from the bottom to the top, and imparting various functionalities (for example, skin health, moisturizing, natural preservation, antibacterial, etc.) to water, and discharging the water to the top, whereby it is possible to perform filtering by using the multiple filters and effectively impart a moisturizing function and additional health functionalities to water and supply the water.

In addition, the present invention provides a beauty water generator having at least one filter container having multiple filters inside a housing and easily mounting and removing the filter container, whereby it is possible to replace an existing filter container with a filter container having multiple filters having various functions and supply beauty water having various functionalities.

Furthermore, the present invention provides a beauty water generator having a relief valve provided at a lower portion of a filter container to regulate an inner pressure and having filters provided inside the filter container and spaced apart a predetermined distance from each other, whereby it is possible to maintain the filtering function and the moisturizing function and supply beauty water.

### BRIEF DESCRIPTION OF THE DRAWINGS

The above and other objects, features and other advantages of the present invention will be more clearly understood from the following detailed description when taken in conjunction with the accompanying drawings, in which:
FIGS. 1 and 2 are views each illustrating a beauty water generator with a high performance filtering function according to an embodiment of the present invention;
FIG. 3 is a view illustrating a mounting portion according to the embodiment of the present invention;
FIGS. 4A and 4B are views each illustrating a first filter portion according to the embodiment of the present invention;
FIGS. 5A and 5C are views each illustrating a second filter portion according to the embodiment of the present invention;
FIG. 6 is a view illustrating a relief valve provided in the first filter part and the second filter part according to the embodiment of the present invention;
FIGS. 7A to 7C are views illustrating multiple sediment filters according to the embodiment of the present invention; and
FIGS. 8A to 8C are views illustrating multiple ion exchange filters according to the embodiment of the present invention.

### DETAILED DESCRIPTION OF THE INVENTION

Advantages and features of the present invention, and method to achieve them will be obvious with reference to embodiments along with the accompanying drawings which are described below. However, it will be understood that present description is not intended to limit the invention to those exemplary embodiments. On the contrary, the invention is intended to cover not only the exemplary embodiments, but also various alternatives, modifications, equivalents, and other embodiments, which may be included within the spirit and scope of the invention as defined by the appended claims. In the detailed description, the same reference numbers of the drawings refer to the same or equivalent parts of the present invention.

In the following description, it is to be noted that, when the functions of conventional elements and the detailed description of elements related with the present invention may make the gist of the present invention unclear, a detailed description of those elements will be omitted. Moreover, terms described below are defined taking into account functions in embodiments of the present invention, which may vary depending on the intent or custom of users and operators. Therefore, definitions of the terms may be determined based on the entire contents of this specification.

Hereinbelow, the embodiments of the present invention will be described in detail with reference to the accompanying drawings.

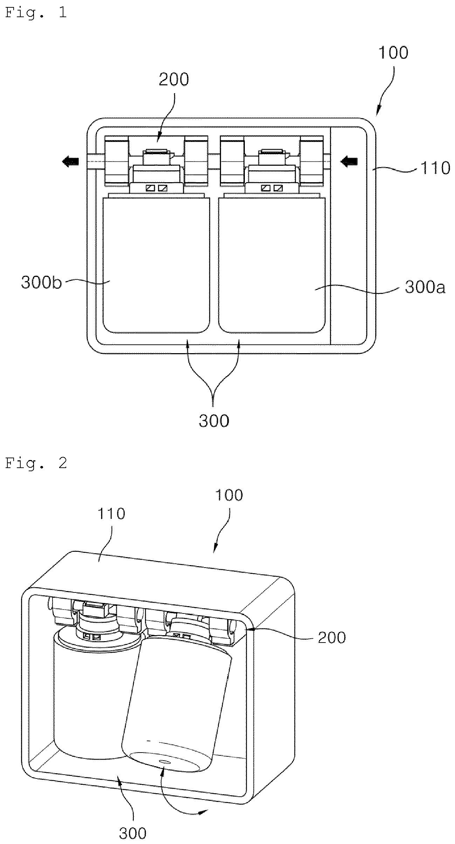

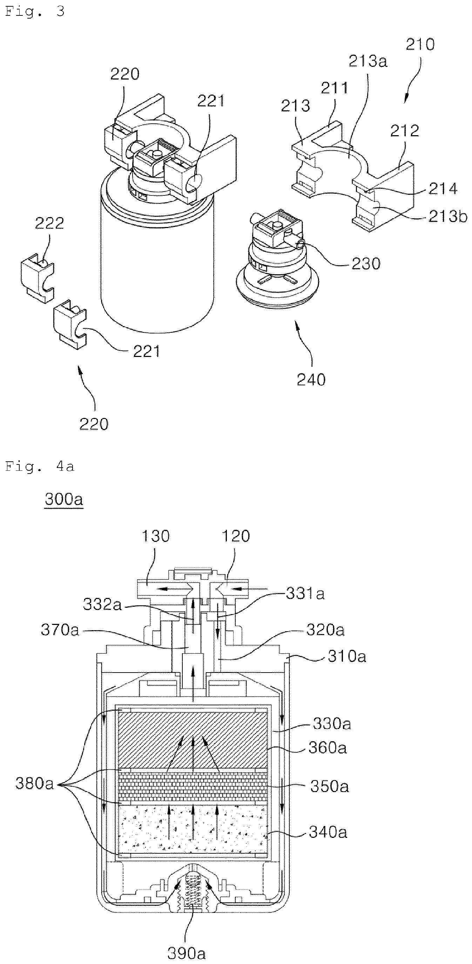

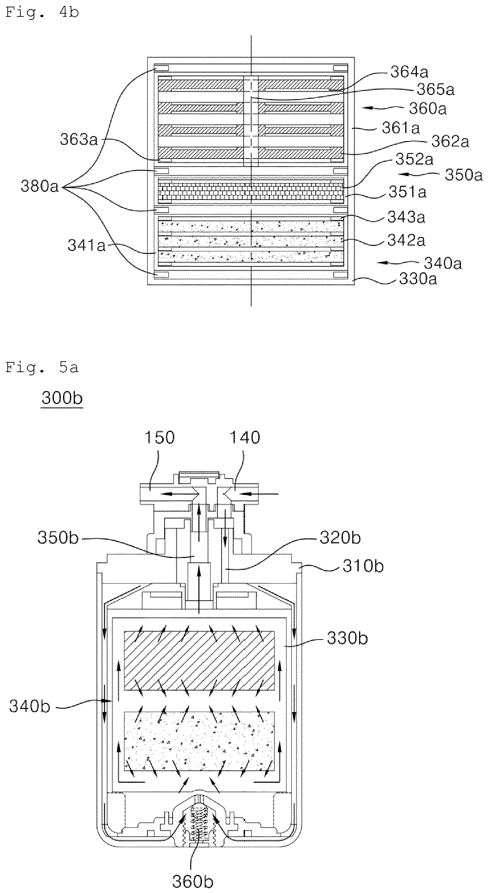

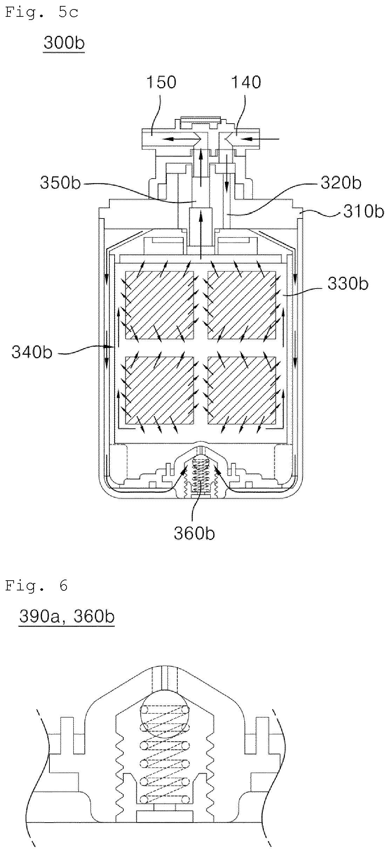

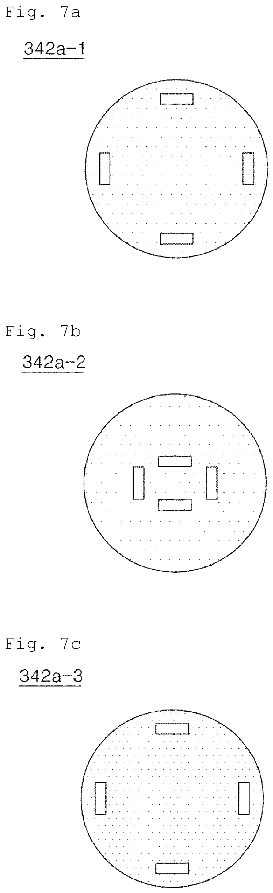

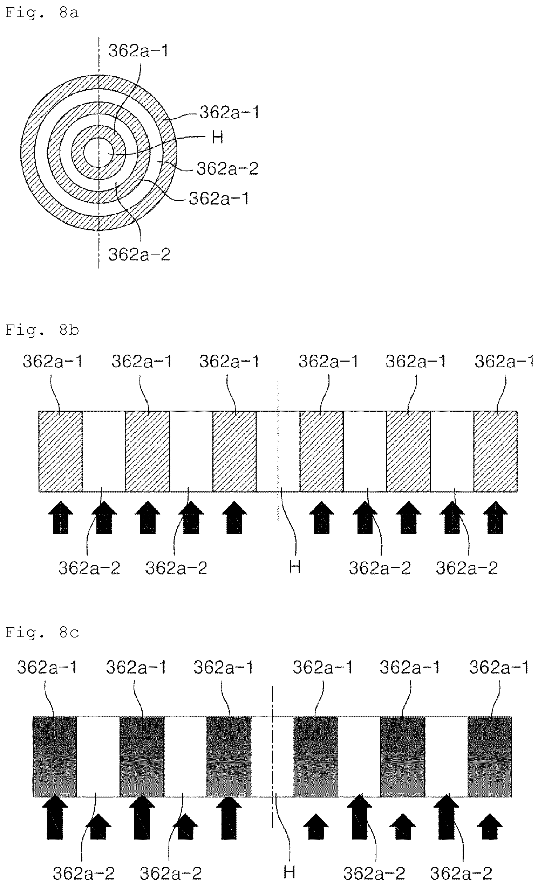

FIGS. 1 and 2 are views each illustrating a beauty water generator with a high performance filtering function according to an embodiment of the present invention; FIG. 3 is a view illustrating a mounting portion according to the embodiment of the present invention; FIGS. 4A and 4B are views each illustrating a first filter portion according to the embodiment of the present invention; FIGS. 5A and 5C are views each illustrating a second filter portion according to the embodiment of the present invention; FIG. 6 is a view illustrating a relief valve provided in the first filter part and the second filter part according to the embodiment of the present invention; FIGS. 7A to 7C are views illustrating multiple sediment filters according to the embodiment of the present invention; and FIGS. 8A to 8C are views illustrating multiple ion exchange filters according to the embodiment of the present invention.

Referring to FIGS. 1 to 3, 4A and 4B, 5A to 5C, 6, 7A to 7C, and 8A to 8C, the beauty water generator with a high performance filtering function according to the embodiment of the present invention includes a housing portion 100, a mounting portion 200, a filter unit 300, and so on.

The housing portion 100 is used as a body of the beauty water generator and includes a housing body 110, a first inflow passage 120, a first outflow passage 130, a second inflow passage 140, a second outflow passage 150, and so on.

Here, the housing body 110 has an accommodating portion therein to accommodate the mounting portion 200 and the filter unit 300. The first inflow passage 120 introduces raw water into a first filter part 300a therethrough, and the first outflow passage 130 discharges purified water from the first filter part 300a therethrough. The second inflow passage 140 introduces the purified water into a second filter part 300b therethrough, and the second outflow passage 150 discharges beauty water from the second filter part 300b therethrough.

The accommodating portion of the housing body 110 may be provided with a front end pipe connection portion at a front end portion of the first inflow passage 120, the front end pipe connection portion being connected to the first inflow passage 120 to supply the raw water. In addition, the accommodating portion of the housing body 110 may be provided with a rear end pipe connection portion at a rear end portion of the second outflow passage 150, the rear end pipe connection portion being connected to a beauty water injecting device (for example, a shower, etc.) provided outside.

A flowmeter (not shown) is provided between the first outflow passage 130 and the second inflow passage 140 in the above-mentioned housing portion 100 to measure a flow rate (quantity) flowing from the first filter part 300a to the second filter part 300b, whereby it is possible to replace a filter in accordance with a predetermined exchange period corresponding to the flow rate.

The mounting portion 200 is provided inside the housing portion 200 in a manner that corresponds to the first filter part 300a and the second filter part 300b. The mounting portion 200 includes a first support member 210, a second support member 220, a rotary shaft cylinder 230, a mounting cap 240, and so on.

Here, the first support member 210 is fixed to an upper portion of the accommodating portion, and the second support member 220 is engaged with the first support member 210 through an insertion recess 214 and an insertion protrusion 222 so as to have a space for axial rotation. The first support member 210 includes a connection plate 213 connecting a first support plate 211 and a second support plate 212 to each other. A rotary recess 213a is formed at a central portion of the connection plate 213 in a semicircular shape (when viewed from the top) to provide a rotary space in which the mounting cap 240 engaged with the rotary shaft cylinder 230 rotates. A first engagement recess 213b having a semicircular shape (when viewed from the side) is formed at both protruding ends in a transverse direction of the connection plate 213 for engagement of the rotary shaft cylinder 230.

In addition, the second support member 220 is engaged with both ends of the connection plate 213 and formed with a second engagement recess 221 having a semicircular shape (when viewed from the side) in the transverse direction for engagement of the rotary shaft cylinder 230. Accordingly, the both ends of the rotary shaft cylinder 230 are engaged in the circular engagement space defined by the first engagement recess 213b and the second engagement recess 221.

The rotary shaft cylinder 230 is provided in the space for axial rotation and has an outflow passage at an outlet end, which corresponds to an inflow passage formed inside an inlet end. As the both ends of the rotary shaft cylinder 230 are stably and rigidly engaged in the circular engagement space defined by the first engagement recess 213b and the second engagement recess 221, the rotary shaft cylinder 230 effectively performs functions thereof such as introducing of the raw water, discharging and introducing of the purified water, and discharging of the beauty water through the first inflow passage 120, the first outflow passage 130, the second inflow passage 140, and the second outflow passage 150.

The mounting cap 240 is rotatably engaged with the rotary shaft cylinder 230, and the first filter part 300a and the second filter part 300b of the filter unit 300 are individually mounted thereon in a rotationally engaged manner. The mounting cap 240 is formed with an engaging hole (not shown) at an upper end portion thereof, in which the rotary shaft cylinder 230 is rotatably engaged. In an opened lower end portion of the mounting cap 240, threads are formed therein to insert and mount the respective filter housings of the filter unit 300 in a rotating manner such that the filter housings can be stably and firmly mounted.

The above-mentioned inflow passage of the rotary shaft cylinder 230 communicates with a first inner inflow passage 320a of the first filter part 300a or a second inner inflow passage 320b of the second filter part 300b. The inflow passage of the rotary shaft cylinder 230 and the first inner inflow passage 320a or the second inner inflow passage 320b are provided such that, when the first filter part 300a or the second filter part 300b is about to be engaged with the mounting cap 240 (that is, when the mounting cap 240 is rotated forward), the flow passages are out of joint and prevented from communicating with each other. Then, when the first filter part 300a or the second filter part 300b is finally mounted on the mounting cap 240 (that is, when the mounting cap 240 is rotated backward and is vertical), the flow passages communicate with each other.

In addition, the outflow passage of the rotary shaft cylinder 230 is provided in a manner similar to that the inflow passage communicates with the first inner inflow passage 320a or the second inner inflow passage 320b in order to communicate with a first inner outflow passage 370a of the first filter part 300a or a second inner outflow passage 350b of the second filter portion 300b.

Furthermore, the inflow passage of the rotary shaft cylinder 230 is further provided therein with a flexible pipe communicating with the first inner inflow passage 320a or the second inner inflow passage 320b such that the inflow passage communicates with the first inner inflow passage 320a or the second inner inflow passage 320b depending on a rotation of the mounting cap 240. Likewise, the outflow passage of the rotary shaft cylinder 230 is further provided therein with a flexible pipe communicating with the first inner outflow passage 370a or the second inner outflow passage 350b such that the outflow passage communicates with the first inner outflow passage 370a or the second inner outflow passage 350b depending on a rotation of the mounting cap 240.

The filter unit 300 has the first filter part 300a that purifies introduced raw water through bottom-up movement and discharges purified water and a second filter part 300b that, when the purified water is introduced therein, imparts functionality to the purified water through bottom-up movement and discharges beauty water. The filter unit 300 is provided inside the housing portion 100 such that the first filter part 300a and the second filter part 300b are mounted and removed by rotating through the mounting portion 200.

Here, the first filter part 300a includes a first filter housing 310a, the first inner inflow passage 320a, a first filter container 330a, a first-first filter 340a, a first-second filter 350a, a first-third filter 360a, a first inner outflow passage 370a, multiple first spacers 380a, a first relief valve 390a, and so on. The first filter part 300a includes the cylindrical first filter housing 310a, and the first filter housing 310a has a thread corresponding to the mounting cap 240 on an outer circumferential surface thereof in order to be inserted into and rotatably mounted on the mounting cap 240.

For example, the first filter part 300a is mounted on the housing portion 100 in a sequential manner such that an upper end portion of the first filter housing 310a is inserted into an opened lower end portion of the rotary shaft cylinder 230 in a state that the mounting cap 240 rotates around the rotary shaft cylinder 230 such that the opened lower end portion faces the front, the upper end portion of the first filter housing 310a is rotated such that the first filter housing 310a is stably and firmly mounted on the mounting cap 240, and then the mounting cap 240 is rotated by pushing the first filter housing 310a backward.

The first inner inflow passage 320a is a passage for transferring raw water to a lower portion along an inner side surface of the first filter housing 310a. The first inner inflow passage 320a is formed along the inner side surface (that is, an inner peripheral surface) of the first filter housing 310a and communicates with the first inflow passage 120 such that raw water flowing through the first inflow passage 120 is transferred to the lower portion of the first filter housing 310a.

The first filter housing container 330a is a cylindrical container provided inside the first filter housing 310a and accommodates the first-first filter 340a, the first-second filter 350a, and the first-third filter 360a. The first filter housing container 330a is provided with a first inlet 331a through which the raw water transferred to the lower portion of the first filter housing 310a flows and is provided with a first outlet 332a at an upper portion thereof, which communicates with the first inner outflow passage 370a and through which purified water is discharged.

The first-first filter 340a is provided in the first filter container 330a and has a first-first filter housing 341a in which multiple sediment filters 342a are stacked. The multiple sediment filters 342a have different pore sizes.

The first-first filter 340a is disposed such that multiple sediment filters 342a provided inside the first-first filter housing 341a are spaced a predetermined distance apart from each other. A first-first inner spacer 343a is provided between each filter of the multiple sediment filters 342a such that the multiple filter segments 342a are spaced a predetermined distance from each other.

Furthermore, the first-first inner spacer 343a is provided at upper and lower inner sides of the first-first filter housing 341a individually such that upper and lower inner surfaces of the first-first filter housing 341a and the multiple sediment filters 342a are spaced a predetermined distance apart.

Here, the multiple sediment filters 342a are provided to filter sediments and pollutants having a pore size of the filter or more. The multiple sediment filters 342a are manufactured using high-density polypropylene or the like, have different pore sizes, and have different passage positions.

For example, as illustrated in FIGS. 7A to 7C, when three sediment filters 342a are provided, a first sediment filter 342a-1 has a pore size of 10 *µ*m, and four passages are formed at edge positions and spaced apart from each other by 90 degrees. A second sediment filter 342a-2 has a pore size of 10 *µ*m, and four passages are formed at center positions and spaced apart from each other by 90 degrees. A third sediment filter 342a-3 has a pore size of 5 *µ*m, and four passages are formed at edge positions and spaced apart from each other by 90 degrees . As a result, by providing the multiple sediment filters 342a having various pore sizes and various passages, it is possible to prevent a flow rate from being reduced due to fouling or the like and to minimize passing of sediments and pollutants.

The first-second filter 350a is provided on the first-first filter 340a and made of a filter using an adsorbent material to adsorb and filter general bacteria, residual chlorine, organic chemicals, and so on. The adsorbent material includes at least one of activated carbon, zeolite, loess, goethite, and illite.

The first-second filter 350a is provided inside a first-second filter housing 351a as a disc-shaped adsorbent filter 352a made of an adsorbent material. The first-second filter 350a is spaced a predetermined distance apart from upper and lower inner surfaces of the first-second filter housing 351a. For this, multiple first-second inner spacers 353a are provided on upper and lower inner side portions of the first-second filter housing 351a.

The first-third filter 360a is provided on the first-second filter 350a and has multiple stacked ion exchange filters (IE filters) 362a. The multiple IE filters 352a include a functional module, which is formed in a disc shape and has an ion exchange function (IE). The functional module is capable of filtering and removing heavy metal ions, hardness-inducing substances, and the like and has functional fibers and filtration fibers being alternately arranged. For example, the multiple IE filters 362a may be provided with multiple functional modules to which a sulfo group is applied for cation exchange.

Here, the multiple IE filters 362a of the first-third filter 360a are stacked inside a first-third filter housing 361a and arranged to be spaced a predetermined distance apart from each other. As the first-third filter housing 361a is provided with first-third inner spacers 363a on upper and lower inner sides respectively, the multiple IE filters 362a are spaced a predetermined distance apart from upper and lower inner surfaces of the first-third filter housing 361a.

In addition, a residual spacer 364a is provided in each portion, which is adjacent to the multiple IE filters 362a.

The first inner outflow passage 370a is provided at a central portion of the first filter part 300a to communicate with the first outflow passage 130 while passing through upper portions of the first filter container 330a and the first filter housing 310a. The first inner outflow passage 370a discharges purified water to the outside of the first filter part 300a through the first-first filter 340a, the first-second filter 350a, and the first-third filter 360a.

The multiple first spacers 380a space the first-first filter 340a, the first-second filter 350a, and the first-third filter 360a apart from each other by a predetermined distance in the first filter container 330a. In addition, the multiple first spacers 380a are provided at upper and lower inner sides of the first filter container 330a such that the filters are spaced a predetermined distance apart from upper and lower inner surfaces of the first filter container 330a.

In addition, the first relief valve 390a is provided at a lower end of the first filter housing 310a, which discharges an inner fluid to maintain a predetermined pressure. The first relief valve 390a is opened and closed to discharge the inner fluid selectively such that it is possible to maintain an internal pressure of the first filter part 300a constant.

For example, as illustrated in FIG. 6, the first relief valve 390a, which is referred to as a safety valve, maintains the internal pressure constant in a manner that an opening is moved to the lower portion while being supported by a spring when a predetermined pressure is reached, and then the spring is restored by elasticity and the opening is raised to the original position and closed when the pressure drops below the predetermined pressure.

The multiple IE filters 362a include functional fibers 362a-1 and filtration fibers 362a-2. The functional fibers 362a-1 and the filtration fibers 362a-2 are alternately arranged at a predetermined interval in a state where the center is provided with a through-hole extending from the upper end to the lower end of the filter. For example, the IE filter 362a includes a through-hole H, a functional fiber 362a-1, a filtration fiber 362a-2, a functional fiber 362a-1, a filtration fiber 362a-2, a functional fiber 362a-1 which are arranged outward in this order.

The filtration process of the IE filter 362a will be further described. As illustrated in FIG. 8B, water passes the filter uniformly and constantly in the initial state of the IE filter 362a. As illustrated in FIG. 8C, when the time elapses, pores are clogged due to adsorption on the functional fibers 362a-1 and the flow rate is decreased. However, the flow rate of the filtration fibers 362a-2 increases such that it is possible to prevent the pressure difference from increasing.

The through-hole H provided in the central portion of the multiple IE filters 362a is provided with a passage post 365a having multiple pores and serving as a passage of introduced water. Water is purified in a manner that, when water flows in from the first-second filter 350a, water passes through the lowermost center through the passage post 365a and flows to a first IE filter 362a where water is ion-exchanged. Thereafter, water flows to a second IE filter 362a located at the upper portion along the passage post 365a and an inner circumferential surface of the flow path post 365a. Then, water is again ion exchanged through the second IE filter 362a and flows to a third IE filter 362a located on the top along the passage post 365a and the inner circumferential surface of the first-third filter housing 361a.

The second filter part 300b includes a second filter housing 310b, the second inner inflow passage 320b, a second filter container 330b, multiple second filters 340b, the second inner outflow passage 350b, a second relief valve 360b, and so on. The second filter part 300b includes the cylindrical second filter housing 310b, and the second filter housing 310b has a thread corresponding to the mounting cap 240 on an outer circumferential surface thereof in order to be inserted into and rotatably mounted on the mounting cap 240.

The second filter part 300b can be stably and firmly mounted on the housing portion 100 in the same manner as that of the first filter part 300a.

The second inner inflow passage 320b is a passage for transferring purified water to a lower portion along an inner side surface of the second filter housing 310b. The second inner inflow passage 320a is formed along the inner side surface (that is, an inner peripheral surface) of the second filter housing 310b and communicates with the second inflow passage 140 such that purified water flowing through the second inflow passage 140 is transferred to the lower portion of the second filter housing 310b.

The second filter container 330b is a cylindrical container provided inside the second filter housing 310b and accommodates the multiple second filters 340b. The second filter housing container 330b is provided with a second inlet 331b through which purified water transferred to the lower portion of the second filter housing 310b flows and is provided with a second outlet 332b at an upper portion thereof, which communicates with the second inner outflow passage 350b and through which functional beauty water is discharged.

The multiple second filters 340b are provided inside the second filter container 330b and impart at least one moisturizing, aroma, vitamin, and antiseptic and antibacterial function to the purified water. The multiple second filters 340b include at least one functional filter selected from the group consisting of a vitamin infusing filter, a moisturizing filter, and an antiseptic and antibacterial filter, wherein the vitamin infusing filter includes vitamin selected from the group consisting of vitamin A, vitamin B, vitamin C, and vitamin E, the moisturizing filter includes a material selected from the group consisting of red ginseng, hempseed oil, honey extract, lotus oil, and camellia oil, and the antiseptic and antibacterial filter includes a material selected from the group consisting of peppermint, lemongrass, chitin, and chamomile.

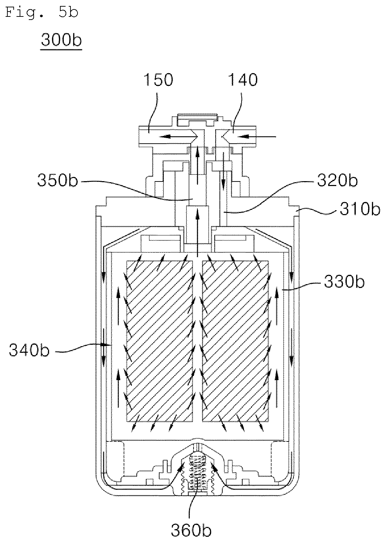

Here, when multiple functional filters are provided, the inside of the cylindrical second filter housing 310b is vertically partitioned or horizontally partitioned as illustrated in FIGS. 5A to 5C. In the case of the structures horizontally partitioned in FIG. 5A, vertically partitioned in FIG. 5B, and horizontally and vertically partitioned in FIG. 5C, when purified water flows through flow passages provided around the filters, multiple discharge holes (not shown) are formed in contact portions of the flow passages, which are in contact with each of the functional filters, to discharge a health functional material continuously. Accordingly, various health functionalities are imparted to flowing purified water.

Although it has been described that two filters horizontally partitioned, vertically partitioned, and horizontally and vertically partitioned are provided as illustrated in FIGS. 5A to 5C, this is illustrative and may be three or more filters horizontally partitioned, vertically partitioned, or horizontally and vertically partitioned. In addition, it is possible to provide an additional functionality, such as adsorption, by further providing a filter containing a ceramic ball between the filters.

The second inner outflow passage 350b is provided at a central portion of the second filter part 300b to communicate with the second outflow passage 150 while passing through upper portions of the second filter container 330b and the second filter housing 310b. The second inner outflow passage 350b discharges functional beauty water to the outside of the second filter part 300b through the multiple second filters 340a.

In addition, the second relief valve 360b is provided at a lower end of the second filter housing 310b, which discharges an inner fluid to maintain a predetermined pressure. The second relief valve 360b is opened and closed to discharge the inner fluid selectively such that it is possible to maintain an internal pressure of the second filter part 300b constant.

Since a configuration of the second relief valve 360b is similar to that of the first relief valve 390a, a detailed description thereof will be omitted.

As a result, the present invention provides a beauty water generator with a high performance filtering function, the beauty water generator not only providing beauty water with various functions such as filtering function using multiple filters and moisturizing function but also maintaining the performance.

In addition, the present invention provides a beauty water generator purifying water introduced into the filter container by using the multiple filters while guiding the water from the bottom to the top, and imparting various functionalities (for example, skin health, moisturizing, natural preservation, antibacterial, etc.) to water, and discharging the water to the top, whereby it is possible to perform filtering by using the multiple filters and effectively impart moisturizing function and additional health functionalities to water and supply the water.

In addition, the present invention provides a beauty water generator having at least one filter container having the multiple filters inside the housing and easily mounting and removing the filter container, whereby it is possible to replace an existing filter container with a filter container having multiple filters having various functions and supply beauty water having various functionalities.

Furthermore, the present invention provides a beauty water generator having the relief valve provided at the lower portion of the filter container to regulate an inner pressure and having the filters provided inside the filter container and spaced apart a predetermined distance from each other, whereby it is possible to maintain the filtering function and the moisturizing function and supply beauty water.

Although the various embodiments of the present invention has been described for illustrative purposes, those skilled in the art will appreciate that various modifications, additions and substitutions are possible, without departing from the scope and spirit of the invention as disclosed in the accompanying claims.

## Claims

1. A beauty water generator with a high performance filtering function, the beauty water generator comprising:
a housing portion used as a body of the beauty water generator;
a mounting portion provided inside the housing portion; and
a filter unit having a first filter part that purifies introduced raw water through bottom-up movement and discharges purified water and a second filter part that, when the purified water is introduced therein, imparts functionality to the purified water through bottom-up movement and discharges beauty water, the filter unit being provided inside the housing portion such that the first filter part and the second filter part are mounted and removed by rotating through the mounting portion.

2. The beauty water generator of claim 1, wherein the housing portion includes:
a housing body having an accommodating portion therein to accommodate the mounting portion and the filter unit;
a first inflow passage through which the raw water is introduced into the first filter part;
a first outflow passage through which the purified water is discharged from the first filter part;
a second inflow passage through which the purified water is introduced into the second filter part; and
a second outflow passage through which the beauty water is discharged from the second filter part.

3. The beauty water generator of claim 2, wherein the mounting portion is provided in a manner that corresponds to the first filter section and the second filter section, and the mounting portion includes:
a first support member fixed to an upper portion of the accommodating portion;
a second support member engaged with the first support member to have a space for axial rotation;
a rotary shaft cylinder provided in the space for the rotational axis and having an outflow passage at an outlet end, which corresponds to an inflow passage formed inside an inlet end; and
a mounting cap which is rotatably engaged with the rotary shaft cylinder and on which the filter unit is mounted in a rotationally engaged manner.

4. The beauty water generator of any one of claims 1 to 3, wherein the first filter part includes:
a cylindrical first filter housing;
a first inner inflow passage transferring the raw water to a lower portion along an inner side surface of the first filter housing;
a first filter container provided inside the first filter housing to accommodate the first filter;
a first-first filter provided inside the first filter container and having multiple sediment filters stacked therein;
a first-second filter provided on the first-first filter and made of an adsorbent material;
a first-third filter provided on the first-second filter and having multiple stacked ion exchange filters (IE filters);
a first inner outflow passage discharging the purified water purified through the first-first filter, the first-second filter, and the first-third filter to the outside of the first filter part; and
multiple first spacers spacing the first-first filter, the first-second filter, and the first-third filter apart from each other by a predetermined distance.

5. The beauty water generator of claim 4, wherein the first filter part further includes:
a first relief valve at a lower end of the first filter housing, which discharges an inner fluid to maintain a predetermined pressure of the first filter part.

6. The beauty water generator of claim 5, wherein the multiple sediment filters have different pore sizes.

7. The beauty water generator of claim 6, wherein the first-first filter is disposed such that the multiple sediment filters provided inside a first-first filter housing are spaced a predetermined distance apart from each other.

8. The beauty water generator of claim 5, wherein the adsorbent material includes at least one of activated carbon, zeolite, loess, goethite, and illite.

9. The beauty water generator of claim 8, wherein the first-second filter is provided inside a first-second filter housing as a disc-shaped filter made of the adsorbent material and spaced a predetermined distance apart from an inner surface of the first-second filter housing.

10. The beauty water generator of claim 5, wherein each of the multiple IE filters has a functional module, and
the functional module is formed in a disc shape, has an ion exchange function (IE), and has functional fibers and filtration fibers being alternately arranged.

11. The beauty water generator of claim 10, wherein the multiple IE filters of the first-third filter are stacked inside a first-third filter housing and arranged to be spaced a predetermined distance apart from each other, and
a residual spacer is provided in each portion, which is adjacent to the multiple IE filters.

12. The beauty water generator of any one of claims 1 to 3, wherein the second filter part includes:
a cylindrical second filter housing;
a second inner inflow passage transferring the purified water to a lower portion along an inner side surface of the second filter housing;
a second filter container provided inside the second filter housing to accommodate the second filter;
multiple second filters provided inside the second filter container and imparting at least one function of moisturizing, aroma, vitamin, and antiseptic and antibacterial to the purified water; and
a second inner outflow passage discharging the beauty water treated through the multiple second filters to the outside of the second filter part.

13. The beauty water generator of claim 12, wherein the second filter part further includes:
a second relief valve at a lower end of the second filter housing, which discharges an inner fluid to maintain a predetermined pressure of the second filter part.

14. The beauty water generator of claim 13, wherein the multiple second filters include at least one functional filter selected from the group consisting of a vitamin infusing filter, a moisturizing filter, and an antiseptic and antibacterial filter,
wherein the vitamin infusing filter includes vitamin selected from the group consisting of vitamin A, vitamin B, vitamin C, and vitamin E,
the moisturizing filter includes a material selected from the group consisting of red ginseng, hempseed oil, honey extract, lotus oil, and camellia oil, and
the antiseptic and antibacterial filter includes a material selected from the group consisting of peppermint, lemongrass, chitin, and chamomile.
